# EUROPÄISCHE PATENTSCHRIFT

(11) **EP 2 839 396 B1**
(45) Veröffentlichungstag und Bekanntmachung des Hinweises auf die Patenterteilung: **17.04.2019**
(21) Anmeldenummer: 13718560.9
(22) Anmeldetag: 19.04.2013
(51) Int. Cl.: G06F 19/00, G06Q 10/10, G16H 50/70, G16H 50/20

(54) **VERFAHREN UND SYSTEM ZUM ERFASSEN VON PROBLEMBILDERN UND ZUM ERMITTELN VON LÖSUNGSVORSCHLÄGEN**
METHOD AND SYSTEM FOR DETECTING PROBLEM IMAGES AND FOR ASCERTAINING SOLUTION PROPOSALS
PROCÉDÉ ET SYSTÈME POUR ENREGISTRER DES TABLEAUX DE PROBLÈMES ET POUR DÉTERMINER DES PROPOSITIONS DE SOLUTIONS

(30) Priorität: 19.04.2012 DE 102012103450
(43) Veröffentlichungstag der Anmeldung: 25.02.2015
(73) Patentinhaber: OMS Software GmbH, 82433 Bad Kohlgrub (DE)
(72) Erfinder: KRAMER, Christian, 81243 München (DE)
(74) Vertreter: 2s-ip Schramm Schneider Bertagnoll Patent- und Rechtsanwälte Part mbB
(86) Internationale Anmeldenummer: PCT/EP2013/058169
(87) Internationale Veröffentlichungsnummer: WO 2013/156596

(56) Entgegenhaltungen:
- WO-A1-02/11035
- WO-A1-2009/083841
- US-A- 4 945 476
- IGOR JURISICA ET AL: "Incremental Iterative Retrieval and Browsing for Efficient Conversational CBR Systems", APPLIED INTELLIGENCE ; THE INTERNATIONAL JOURNAL OF ARTIFICIALINTELLIGENCE, NEURAL NETWORKS, AND COMPLEX PROBLEM-SOLVING TE, KLUWER ACADEMIC PUBLISHERS, BO, Bd. 12, Nr. 3, 1. Mai 2000 (2000-05-01), Seiten 251-268, XP019204460, ISSN: 1573-7497, DOI: 10.1023/A:1008375309626
- COOPER J W ET AL: "OBIWAN-a visual interface for prompted query refinement", SYSTEM SCIENCES, 1998., PROCEEDINGS OF THE THIRTY-FIRST HAWAII INTERNA TIONAL CONFERENCE ON KOHALA COAST, HI, USA 6-9 JAN. 1998, LOS ALAMITOS, CA, USA,IEEE COMPUT. SOC, US, Bd. 2, 6. Januar 1998 (1998-01-06), Seiten 277-285, XP010262890, DOI: 10.1109/HICSS.1998.651710 ISBN: 978-0-8186-8255-1

## Beschreibung

### Gebiet der Erfindung

Die Erfindung betrifft ein Verfahren zum Erfassen von Problembildern und zum Ermitteln passender Lösungsvorschläge.

### Hintergrund der Erfindung und Stand der Technik

Im Stand der Technik sind Diagnosesysteme bekannt, die auf einer computergestützten Datenbanksuche beruhen, bei denen ein Nutzer ein oder mehrere konkret festgestellte Probleme in Textform eingeben kann, woraufhin das Diagnosesystem zu den eingegebenen Problemen passende Diagnosen und passende Lösungsvorschläge ermittelt.

Derartige Diagnosesysteme sind allerdings relativ unzulänglich und ungenau, da sie letztlich darauf beruhen, die in Textform eingegebene Problembeschreibung zu analysieren und anhand des Ergebnisses der Analyse entsprechende Diagnosen zu finden. Eine fehlerhafte oder ungenaue Analyse der eingegebenen textuellen Problembeschreibung kann zu fehlerhaften Diagnosen führen, was wiederum dazu führen kann, dass das System falsche oder unpassende Lösungsvorschläge ermittelt. Ferner hängen die ermittelten Lösungsvorschläge erheblich von der Qualität der in Textform eingegebenen Problembeschreibung ab.

Ein weiterer Nachteil bekannter Systeme besteht darin, dass diese lediglich von Fachpersonal, bei medizinischen Diagnosesystemen etwa von Ärzten oder Medizinern bedient werden können, da für eine möglichst exakte Diagnose eine möglichst umfassende und exakte Problembeschreibung, etwa Beschreibung der Symptome notwendig ist. Nicht ausgebildetes Personal kann ein solches aus dem Stand der Technik bekanntes Diagnosesystem daher nicht oder nur unzureichend nutzen, um etwa selbst eine Diagnose durchzuführen und nach geeigneten Lösungen zu recherchieren.

Ein weiterer Nachteil bekannter Diagnosesysteme besteht darin, dass, sofern das Diagnosesystem eine Maßnahme zur Lösung eines Problems vorschlägt, der Erfolg der Maßnahme für zukünftige Diagnosen und der damit verbundenen Maßnahmen nicht berücksichtigt wird, weil es sich bei den bekannten Diagnosesystemen um im Wesentlichen statische Systeme handelt, bei denen einem bestimmten Problem eine oder mehrere Diagnosen zugeordnet sind, wobei einer Diagnose wiederum ein oder mehrere Lösungsvorschläge zugeordnet sind. Eine Anpassung kann nur dadurch erfolgen, indem in dem betreffenden Diagnosesystem manuell neue Probleme hinterlegt werden, den neuen oder den bestehenden Problemen neue oder geänderte Lösungen zugeordnet werden bzw. bereits zugeordnete Lösungen entfernt werden. Diese manuelle Anpassung hat den erheblichen Nachteil, dass potentiell erfolgversprechende Lösungen zu bestimmten Problemen unberücksichtigt bleiben können und daher als mögliche Lösungsansätze für bestimmte Probleme nicht zur Verfügung stehen,

Die Druckschrift WO 2009/083841 offenbart ein solches bekanntes Diagnosesystem und - verfahren, in dem Lösungsvorschläge anhand von durch einen Benutzer eventuell iterativ ausgewählten Parametern von Problembildern erstellt werden, wobei das in der Druckschrift beschriebene System und Verfahren nicht als netzwerktauglich angesehen werden kann, keine Rückmeldung über den Erfolg des vorgeschlagenen Lösungsvorschlags vermittelt und insbesondere keine Gewichtung der Lösungsvorschläge vorsieht.

### Aufgabe der Erfindung

Aufgabe der vorliegenden Erfindung ist es daher, Lösungen bereitzustellen, mit welchen die Erfassung von Problembildern besonders einfach und insbesondere auch durch einen Laien möglich ist und mit welchen geeignete Lösungsvorschläge auch bei sehr komplexen Problembildern ermittelt werden können. Eine weitere Aufgabe der Erfindung ist darin zu sehen, Lösungen bereitzustellen, welche es ermöglichen, bei der Auswahl eines oder mehrerer Lösungsvorschläge die Ergebnisse bereits angewandter bzw. durchgeführter Lösungen zu berücksichtigen.

### Erfindungsgemäße Lösung

Diese Aufgabe wird erfindungsgemäß durch ein Verfahren zum Erfassen von Problembildern und zum Ermitteln von Lösungsvorschlägen nach den unabhängigen Ansprüchen gelöst. Vorteilhafte Ausgestaltungen und Weiterbildungen der Erfindung sind in den jeweiligen abhängigen Ansprüchen angegeben.

Bereitgestellt wird demnach ein Verfahren zum Erfassen von Problembildern und zum Ermitteln von Lösungsvorschlägen in einem System umfassend zumindest eine Servereinrichtung und zumindest eine Clienteinrichtung, wobei die Clienteinrichtung über ein Kommunikationsnetzwerk mit der Servereinrichtung koppelbar ist, wobei die Servereinrichtung mit einer Speichereinrichtung gekoppelt ist, in welcher eine Anzahl von Problembildern, eine Anzahl von Parametern und eine Anzahl von Lösungen gespeichert sind, wobei den Problembildern jeweils zumindest ein Parameter und zumindest eine Lösung zugeordnet sind, wobei die einem Problembild zugeordneten Parameter das Problembild beschreiben, und wobei die Servereinrichtung
a) eine Anzahl von Parametern an die Clienteinrichtung überträgt, zur Auswahl eines Parameters durch einen Nutzer der Clienteinrichtung,
b) den ausgewählten Parameter von der Clienteinrichtung empfängt,
c) aus den gespeicherten Problembildern jene Problembilder auswählt, denen die empfangenen Parameter zugeordnet sind, und aus den den ausgewählten Problembildern zugeordneten Parametern jene Parameter auswählt, welche nicht den bereits empfangenen Parametern entsprechen,
d) die ausgewählten Parameter zur Auswahl an die Clienteinrichtung überträgt;
e) die Schritte b) bis d) solange wiederholt bis in dem Schritt c) keine Parameter mehr ausgewählt werden können oder bis die Servereinrichtung von der Clienteinrichtung ein Terminierungssignal empfängt,
f) die den ausgewählten Problembildern zugeordneten Lösungen ermittelt und als Lösungsvorschläge an die Clienteinrichtung übertragt, zur Auswahl eines Lösungsvorschlages durch den Nutzer der Clienteinrichtung, und
g) von der Clienteinrichtung eine Nachricht empfängt, welche eine Information darüber umfasst, ob die Anwendung des durch den Nutzer ausgewählten Lösungsvorschlages auf das Problembild erfolgreich war.

Bei einem Problembild handelt es sich demnach um ein Problem (z.B. eine vermeintliche Krankheit), das durch einen oder mehrere Parameter (z.B. Symptome) beschrieben bzw. definiert wird. Bei einem Problembild handelt es sich nicht um eine Computergraphik oder dergleichen.

Weil die Beschreibung des Problembildes durch Auswahl von Parametern erfolgt, wird vermieden, dass ein Nutzer des Systems zur Beschreibung des Problembildes textuelle Eingaben machen muss, was die Bedienbarkeit des Systems erheblich verbessert und wesentlich detailliertere und genauere Problembeschreibungen ermöglicht. Insbesondere wird vermieden, dass eine semantische Analyse eines Textes, mit das Problembild beschrieben wird, vorgenommen werden muss, die bekanntermaßen mit einer gewissen Ungenauigkeit behaftet ist und die einen erheblichen Rechenaufwand erfordert.

Die Servereinrichtung kann in einem weiteren Schritt den durch den Nutzer ausgewählten Lösungsvorschlag von der Clienteinrichtung empfangen, den empfangenen Lösungsvorschlag zusammen mit einer Nutzerkennung in der Speichereinrichtung speichern, und dem gespeicherten Lösungsvorschlag die empfangenen Parameter zuordnen.

Die Servereinrichtung empfängt von der Clienteinrichtung zumindest eine Statusnachricht welche eine Information darüber umfasst, ob und wie sich die Parameter durch die Anwendung des ausgewählten Lösungsvorschlages verändern.

Vorteilhaft ist es, wenn die zumindest eine Statusnachricht in vorbestimmten zeitlichen Abständen empfangen wird. Damit kann eine Änderung der Parameter über die Zeit dokumentiert werden.

Die Änderung der Parameter über die Zeit wird in der Speichereinrichtung gespeichert und dem ausgewählten Lösungsvorschlag zugeordnet, wobei basierend auf der Änderung der Parameter über die Zeit für den ausgewählten Lösungsvorschlag ein Gewichtungsfaktor ermittelt wird und dem ausgewählten Lösungsvorschlag zugeordnet wird, wobei vorzugsweise beim Ermitteln des Gewichtungsfaktors ein bereits dem ausgewählten Lösungsvorschlag zugeordneter Gewichtungsfaktor berücksichtigt wird. Aus der Änderung der Parameter über die Zeit kann das System selbständig ermitteln, ob der von dem System vorgeschlagene Lösungsvorschlag zielführend ist, und dem Nutzer des Systems gegebenenfalls einen alternativen Lösungsvorschlag vorschlagen. Durch Vorsehen eines Gewichtungsfaktors kann die "Qualität" eines Lösungsvorschlages für ein bestimmtes Problembild ermittelt werden, sodass das System über die Zeit für ein bestimmtes Problem immer bessere Lösungen ermitteln und den Nutzern vorschlagen kann. Das System ist in dieser Hinsicht "selbstlernend".

In dem Schritt f) kann für die an die Clienteinrichtung zu übertragenden Lösungsvorschläge eine Reihenfolge ermittelt wird, welche die Gewichtungsfaktoren der Lösungsvorschläge berücksichtigt.

Zusätzlich zu den im Schritt c) ausgewählten Parametern können weitere Parameter ausgewählt werden, welche nicht den ausgewählten Problembildern zugeordnet sind, und als optionale Parameter zur Auswahl an die Clienteinrichtung übertragen werden, insbesondere dann, wenn in dem Schritt c) keine Parameter mehr ausgewählt werden können.

Die optionalen Parameter können auf Anforderung durch die Clienteinrichtung ausgewählt und übertragen werden.

Bei einer Auswahl eines optionalen Parameters durch den Nutzer kann ein neues Problembild in der Speichereinrichtung gespeichert werden und dem neuen Problembild können die empfangenen Parameter zugeordnet werden.

In dem Schritt f) können Lösungen ermittelt werden, bei denen den entsprechenden Problembildern Parameter zugeordnet sind, welche zumindest teilweise von empfangenen Parametern umfasst sind, und als Lösungsvorschläge an die Clienteinrichtung übertragen werden. Damit können Lösungen vorgeschlagen werden, denen nur einige der ausgewählten Parameter zugeordnet sind. Aus den auf diese Art bereitgestellten Lösungen können im weiteren zeitlichen Verlauf neue konkrete Problemlösungen für neue Problembilder erzeugt werden.

Die Statusnachricht kann zusätzlich eine weitere Information darüber umfassen, ob zu den bereits empfangenen Parametern weitere Parameter hinzugekommen sind, wobei die weiteren Parameter von der Servereinrichtung empfangen werden.

Die weiteren Parameter können dem neuen Problembild zugeordnet werden.

Alternativ oder zusätzlich kann ein neues Problembild in der Speichereinrichtung gespeichert werden und dem neuen Problembild zumindest die weiteren Parameter zugeordnet werden.

Der dem ausgewählten Lösungsvorschlag zugeordnete Gewichtungsfaktor kann neu ermittelt werden, wobei die Information darüber, dass zu den bereits empfangenen Parametern weitere Parameter hinzugekommen sind, den neu zu ermittelnden Gewichtungsfaktor vorzugsweise negativ beeinflussen.

In der konkreten Ausgestaltung der Erfindung umfassen die Problembilder Krankheitsbilder und die Parameter umfassen Symptome, mit denen Krankheitsbilder beschrieben werden können, und wobei die Lösungsvorschläge Therapievorschläge zur Behandlung zumindest eines Krankheitsbildes umfassen.

### Kurzbeschreibung der Figuren

Weitere Einzelheiten und Merkmale der Erfindung ergeben sich aus der nachfolgenden Beschreibung in Verbindung mit der Zeichnung. Es zeigt:
- Fig. 1: ein erfindungsgemäßes System zum Erfassen von Problembildern und zum Ermitteln von Lösungsvorschlägen mit einer Anzahl von Clienteinrichtungen, welche über ein Kommunikationsnetzwerk mit einer Servereinrichtung koppelbar sind;
- Fig. 2: ein Ablaufdiagramm eines erfindungsgemäßen Verfahrens zum Erfassen von Problembildern und zum Ermitteln von Lösungsvorschlägen;
- Fig. 3: ein konkretes Beispiel zum Ermitteln von Lösungsvorschlägen; und
- Fig. 4: ein vereinfachtes Sequenzdiagramm zur Verdeutlichung der dynamischen Anpassbarkeit des erfindungsgemäßen Systems.

### Detaillierte Beschreibung der Erfindung

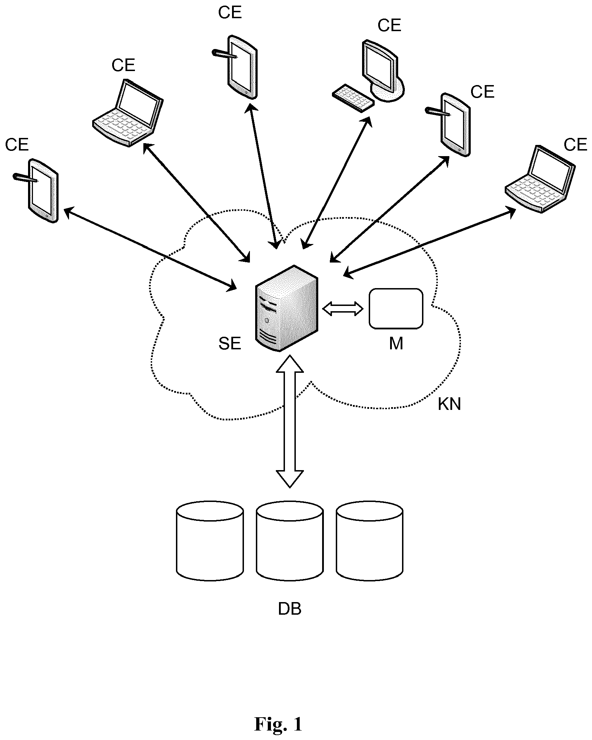

**Fig. 1** zeigt ein System zum Erfassen von Problembildern und zum Ermitteln von Lösungen bzw. Lösungsvorschlägen zu einem erfassten Problembild.

Das System umfasst eine Servereinrichtung SE, welche über ein Kommunikationsnetzwerk KN mit einer Anzahl von Clienteinrichtungen CE koppelbar ist. Das Kommunikationsnetzwerk KN kann beispielsweise ein Mobilfunknetz oder das Internet sein. Bei dem Kommunikationsnetzwerk KN kann es sich aber auch um ein lokales Netzwerk (LAN) handeln. Die Servereinrichtung SE ist mit einer Speichereinrichtung gekoppelt, welche eine oder mehrere Datenbanken DB umfassen kann. Die Speichereinrichtung kann auch Bestandteil der Servereinrichtung SE sein.

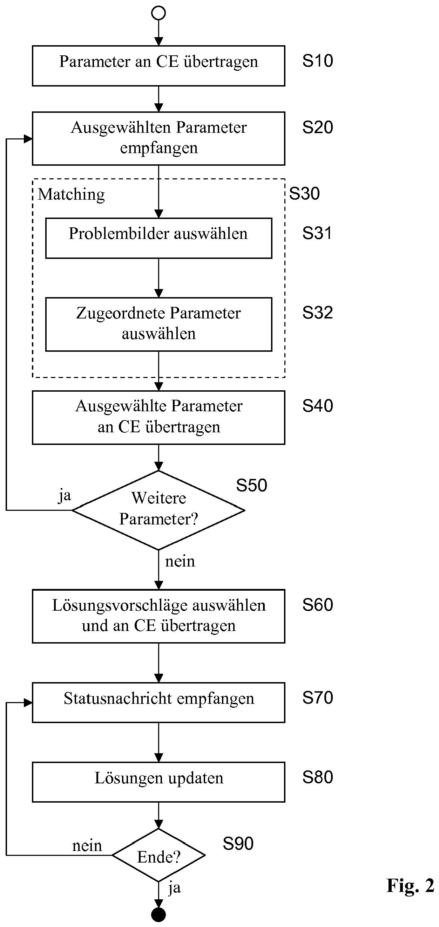

Ferner ist die Servereinrichtung SE mit einem sogenannten Matching-Modul M gekoppelt, welches angepasst ist, zu einem oder mehreren erfassten Parametern weitere mögliche zu erfassende Parameter oder eine Anzahl möglicher Lösungsvorschläge zu ermitteln, welche den Clienteinrichtungen CE bereitgestellt werden können. Das Matching-Modul kann durch eine weitere Servereinrichtung implementiert sein oder Bestandteil der Servereinrichtung SE sein. Die Funktionsweise des Matching-Moduls M wird mit Bezug auf Fig. 2 bis Fig. 4 näher beschrieben.

In der Speichereinrichtung bzw. in den Datenbanken DB sind eine Anzahl von Problembildern, eine Anzahl von Parametern und eine Anzahl von Lösungen bzw. Lösungsvorschlägen gespeichert, wobei den Problembildern jeweils zumindest ein Parameter und zumindest eine Lösung bzw. Lösungsvorschlag zugeordnet sind. Im Bereich der Medizin kann ein Problembild beispielsweise ein Krankheitsbild sein, welches durch eine Anzahl von Symptomen (Parameter) beschrieben wird.

Eine Lösung bzw. ein Lösungsvorschlag kann im Bereich der Medizin eine therapeutische Maßnahme sein, welche vorgesehen ist, die das Krankheitsbild definierenden Symptome auf ein gewünschtes Normalmaß zu bringen.

Ferner ist die Speichereinrichtung bzw. die Datenbanken DB vorgesehen, um neue Problembilder, Parameter und Lösungen bzw. Lösungsvorschläge zu speichern, welche sich durch ein Feedback der Nutzer des erfindungsgemäßen Systems ergeben können. Beispielsweise kann ein Nutzer zu einem bestimmten Problembild die Änderung der das Problembild beschreibenden Parameter über die Zeit dokumentieren, was Rückschlüsse über den Erfolg des angewandten Lösungsvorschlages zulässt.

Im Bereich der Medizin kann beispielsweise ein Nutzer für ein bestimmtes Krankheitsbild und eine bestimmte angewandte Therapie angeben, wie sich die Symptome im Verlauf der Therapiemaßnahme verändern. Sind zu einem bestimmten Krankheitsbild mehrere Therapiemaßnahmen möglich, kann aus den jeweiligen Veränderungen der Symptome auf die Qualität der Therapiemaßnahme im Vergleich zu den anderen möglichen Therapiemaßnahmen geschlossen werden.

Zudem ist es möglich, dass die Anwendung einer bestimmten Therapiemaßnahme dazu führt, dass neue Symptome auftreten, welche dann ebenfalls als Ergebnis der Therapiemaßnahme in der Datenbank DB gespeichert werden. Dadurch kann es auch vorkommen, dass neue noch nicht in der Datenbank gespeicherte Krankheitsbilder entstehen, welche dann ebenfalls in den Datenbanken DB gespeichert werden können. Gleichzeitig können zu den neuen Krankheitsbildern, für die in der Datenbank noch keine Therapien gespeichert sind, automatisch neue Therapien bzw. Therapievorschläge angelegt werden, welche sich daraus ergeben, dass der Nutzer des Systems eine oder mehrere angewandte Therapiemaßnahmen und die damit verbundenen Änderungen der Symptome über die Zeit angibt. Damit wird ein im Wesentlichen selbstlernendes System bereitgestellt, welches über die Zeit automatisch neue Krankheitsbilder und die hierfür möglichen Therapien erkennen und in der Speichereinrichtung speichern kann, sowie bestehende Therapiemaßnahmen automatisch optimieren kann.

Mit der Servereinrichtung SE kann eine sehr große Anzahl von Clienteinrichtungen gekoppelt sein, sodass wiederum eine sehr große Anzahl von Nutzern Problembilder erfassen und den Erfolg der hierfür vorgeschlagenen Lösungsvorschläge hinterlegen können. Durch diese große Anzahl von Nutzern ist es einerseits möglich Ausreißer zu erkennen, etwa wenn zu einem bestimmten Problembild nur sehr wenige Nutzer eine bestimmte Lösung als erfolgreich angegeben haben, während eine sehr große Anzahl von Nutzern für das selbe Problembild eine andere Lösung als erfolgreich angegeben haben. Andererseits wird es möglich, aufgrund der sehr großen Anzahl von Nutzern zu ermitteln, welche Lösungen bzw. Lösungsvorschläge bei welchen Problembildern zu welchen Änderungen der Parameter geführt hat und welche Lösungen bzw. Lösungsvorschläge am erfolgreichsten waren. Die zu einem bestimmten Problembild, etwa ein Krankheitsbild ermittelten Lösungsvorschläge, etwa Therapievorschläge, werden über die Zeit immer genauer, weil beim Ermitteln der Lösungsvorschläge immer mehr Informationen der Nutzer mit einfließen (sogenannte Schwarmintelligenz).

**Fig. 2** zeigt ein Ablaufdiagramm eines erfindungsgemäßen Verfahrens zum Erfassen von Problembildern und zum Ermitteln von Lösungsvorschlägen. Das erfindungsgemäße Verfahren wird nachfolgend anhand eines Beispieles aus der Medizin näher beschrieben. Das erfindungsgemäße Verfahren kann aber auch auf andere Problemdomänen angewandt werden. Beispielsweise kann das erfindungsgemäße Verfahren eingesetzt werden, um ein System zur Lösung von Problemen bei Automobilen oder zum Lösen von Problemen bei der Blumenzucht oder bei der Tierhaltung bereitzustellen. Das Verfahren kann auch eingesetzt werden, um ein System zur Lösung von Problemen im Bereich des Mobilfunkes bereitzustellen. Beispielsweise kann ein Mobilfunkbetreiber oder ein Mobilfunkausrüster ein erfindungsgemäßes Verfahren implementieren, mit dem unter Angabe von Parametern bestimmte Problembilder erfasst werden können und zu den erfassten Problembildern Lösungsvorschläge ermittelt werden können, wobei die Auswirkungen der Anwendung einer Lösungen auf die Parameter dokumentiert werden können, um so auf den Erfolg der Lösung schließen zu können oder automatisch neue Problembilder oder neue Lösungsvorschläge generieren zu können.

In einem ersten Schritt S10 überträgt die Servereinrichtung SE eine Anzahl von Parametern an eine Clienteinrichtung CE. An der Clienteinrichtung CE werden die übertragenen Parameter an einer Anzeigeeinrichtung zur Auswahl eines Parameters durch einen Nutzer der Clienteinrichtung CE angezeigt. Die Clienteinrichtung CE kann ein herkömmlicher Computer, ein Mobiltelefon, ein Tablet-PC, ein Smartphone, oder dergleichen sein. Im medizinischen Umfeld kann es sich bei den übertragenen Parametern um Symptome handeln, mit denen ein Krankheitsbild beschrieben bzw. näher spezifiziert werden kann.

Der Nutzer kann an der Clienteinrichtung einen Parameter aus der Menge der übertragenen Parameter auswählen, wobei der ausgewählte Parameter in dem Schritt S20 an die Servereinrichtung SE übertragen und dort empfangen wird. Indem die Servereinrichtung SE mögliche auszuwählende Parameter an die Clienteinrichtung CE überträgt, wird vermieden, dass ein Nutzer des Systems zur Beschreibung der Symptome eines Krankheitsbildes textuelle Eingaben machen muss, was die Bedienbarkeit des Systems erheblich verbessert. Ferner wird dadurch vermieden, dass aufgrund von fehlerhaften Texteingaben unpassende oder falsche Therapien ermittelt werden. Ein weiterer Vorteil bei der Auswahl von Parametern (die Auswahl kann beispielsweise über sogenannte Checkboxen erfolgen) können die Parameter bzw. die Symptome in einer auch für einen Laien verständlichen Sprache angezeigt werden, weil in diesem Fall von dem erfindungsgemäßen System der Text des Parameters bzw. des Symptoms nicht mehr ausgewertet werden muss. Zudem kann dadurch die Beschreibung eines Problembildes bzw. eines Krankheitsbildes wesentlich effizienter und zeitsparender erfolgen.

In dem medizinischen Umfeld können Parameter Symptome sein, beispielsweise hoher Blutdruck, sehr hoher Blutdruck, Halsschmerzen, Fieber, hohes Fieber, sehr hohes Fieber oder dergleichen.

Nach dem Empfang des ausgewählten Parameters bzw. des ausgewählten Symptoms in dem Schritt S20 wird serverseitig in einem Schritt S30 ein sogenanntes Matching durchgeführt, mit dem weitere Parameter bzw. Symptome ermittelt werden, welche zu dem in dem Schritt S20 empfangenen Parametern passen. Hierfür werden zunächst in dem Schritt S31 jene Problembilder bzw. Krankheitsbilder ausgewählt, welchen die in dem Schritt S20 empfangenen Parametern zugeordnet sind. Damit scheiden zur weiteren Bearbeitung all jene Krankheitsbilder bzw. Problembilder aus, welche diesen Parameter nicht enthalten.

In einem weiteren Schritt S32 werden zu den im Schritt S31 ausgewählten Problembilder bzw. Krankheitsbildern jene Parameter bzw. Symptome ermittelt, welche nicht den in den Schritt S20 übertragenen Parametern bzw. Symptomen entsprechen. Dies kann beispielsweise durch einfache Differenzbildung erfolgen.

Die in dem Schritt S32 ermittelten Parameter bzw. Symptome werden in einem weiteren Schritt S40 an die Clienteinrichtung CE übertragen und dort zur Auswahl durch den Nutzer zur Anzeige gebracht. Dadurch verringert sich auf der Nutzerseite die Anzahl der möglichen auszuwählenden Parameter bzw. Symptome, denn es werden nur mehr solche Symptome zur Auswahl angeboten, welche in Kombination mit dem bereits in dem Schritt S20 übertragenen Parametern zu einem Problembild bzw. Krankheitsbild passen.

Die Schritte S20 bis S40 können solange wiederholt werden, bis in dem Schritt S32 keine weiteren auszuwählenden Parameter mehr ermittelt werden können oder bis die Servereinrichtung von der Clienteinrichtung ein Terminierungssignal empfängt.

Sind in dem Schritt S40 weitere mögliche Parameter an die Clienteinrichtung CE übertragen worden, kehrt das Verfahren zu dem Schritt S20 zurück, in welchem ein weiterer durch einen Nutzer ausgewählter Parameter empfangen wird. In der zweiten Iteration hat die Servereinrichtung SE von der Clienteinrichtung CE zwei ausgewählte Parameter empfangen, welche in dem nachfolgenden Matching-Schritt S30 berücksichtigt werden. Dies führt dazu, dass in der zweiten Iteration die Anzahl möglicher weiterer auszuwählender Parameter nochmals reduziert wird. Die in den Schritt S40 zu übertragenden Parameter reduzieren sich damit ebenfalls, sodass die Anzahl der an der Clienteinrichtung zur Auswahl angezeigtem Parameter mit jedem Iterationsschritt vorzugsweise kleiner wird.

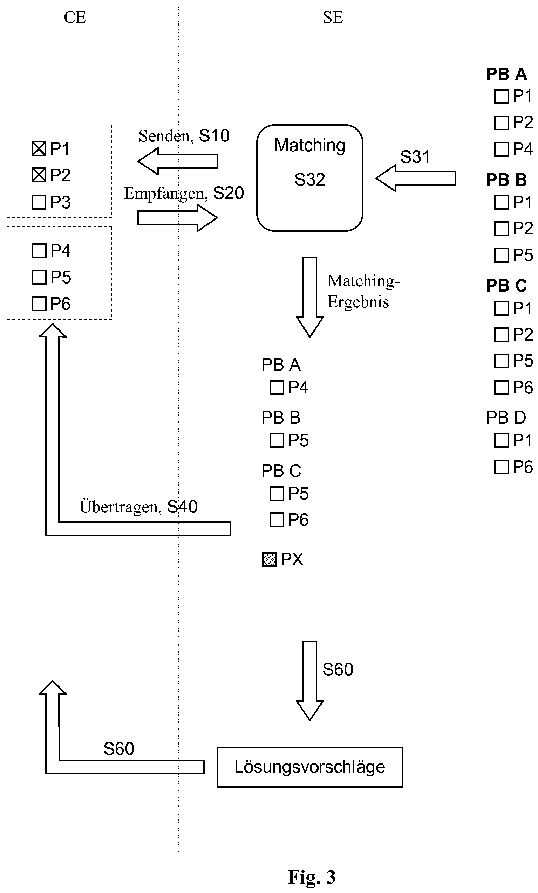

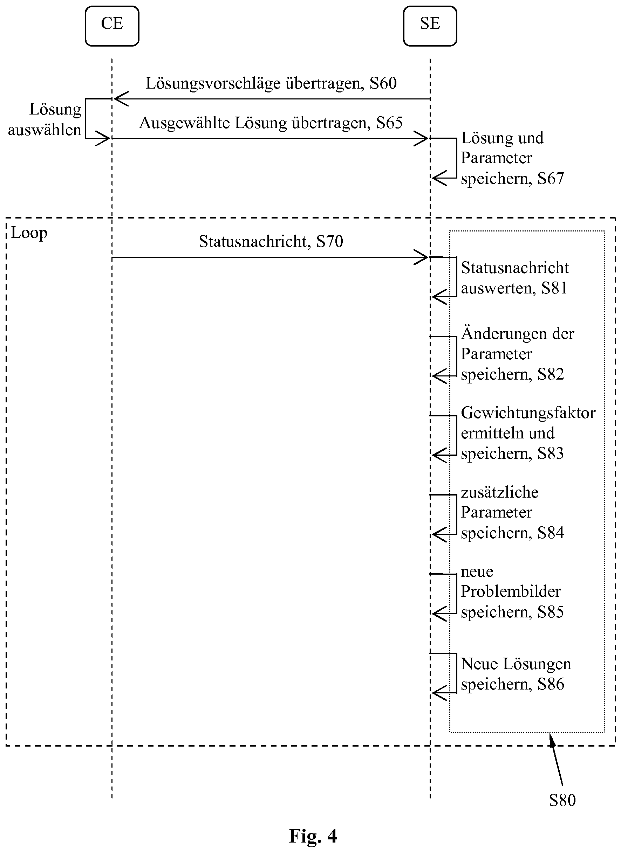

Das System unterstützt einen Nutzer bei der Beschreibung bzw. Definition eines Problembildes bzw. Krankheitsbildes, indem es den Nutzer gezielt durch Reduzierung der möglichen Parameter zu einem bekannten Krankheitsbild hinführt. Die Möglichkeit der Eingabe von Parametern bzw. Symptomen, welche dem Benutzer nicht zur Auswahl angeboten werden, wird mit Bezug auf Fig. 3 und Fig. 4 näher beschrieben.

Nachdem in dem Schritt S32 keine weiteren Parameter zur Übertragung an die Clienteinrichtung mehr ausgewählt werden können oder nachdem die Servereinrichtung von der Clienteinrichtung ein Terminierungssignal empfangen hat, ermittelt die Servereinrichtung in dem Schritt S60 für die empfangenen Parameter bzw. für die zu den empfangenen Parametern passenden Krankheitsbildern Lösungsvorschläge bzw. Therapievorschläge und überträgt diese zur Auswahl durch den Nutzer an die Clienteinrichtung CE. Werden durch die Servereinrichtung mehrere Lösungsvorschläge bzw. Therapievorschläge ermittelt, kann der Nutzer an der Clienteinrichtung CE einen der möglichen Therapievorschläge auswählen.

Die Auswahl eines Therapievorschlages wird an die Servereinrichtung übermittelt und dort zusammen mit einer eindeutigen Nutzerkennung in der Speichereinrichtung gespeichert. Für die Authentifizierung bzw. Autorisierung eines bestimmten Nutzers können aus dem Stand der Technik bekannte Verfahren herangezogen werden.

Weil der ausgewählte Therapievorschlag zusammen mit einer eindeutigen Nutzerkennung gespeichert ist, wird es dem Nutzer ermöglicht, die Auswirkungen der ausgewählten Therapie auf den Krankheitsverlauf bzw. auf die Symptome über die Zeit zu dokumentieren. Der Nutzer kann hierzu beispielsweise an der Clienteinrichtung CE in vorbestimmten zeitlichen Abständen Veränderungen an den Symptomen bzw. Parametern eingeben. Diese Veränderungen werden in einer Statusnachricht in dem Schritt S70 an die Servereinrichtung übertragen und dort empfangen. Anhand der empfangenen Statusnachricht bzw. anhand der Änderungen an den Parametern kann die Servereinrichtung eine Bewertung der ausgewählten Therapie vornehmen, wie mit Bezug auf Fig. 4 näher beschrieben wird. Aufgrund der Bewertung kann in einem weiteren Schritt S80 die ausgewählte Lösung bzw. die ausgewählte Therapie aktualisiert werden, sodass für zukünftig erfasste Krankheitsbilder die aktualisierte Therapie bei der Auswahl in dem Schritt S60 zur Verfügung steht.

Ergibt die Statusnachricht, dass sich die Parameter bzw. Symptome verschlechtert haben, kann der ausgewählten Therapie ein negativer Bewertungsfaktor zugeordnet werden. Ergibt die Auswertung der Statusnachricht, dass sich die Parameter bzw. Symptome verbessert haben, kann der ausgewählten Therapie ein positiver Bewertungsfaktor zugeordnet werden.

Wird das System von einer sehr großen Anzahl Nutzern genutzt, werden Aussagen über den Erfolg einer Therapie immer genauer und spezifischer und können zudem in einer bestimmten Reihenfolge dem Nutzer zur Auswahl angeboten werden. Der Schwarmansatz bzw. die Ausnutzung der Schwarmintelligenz einer großen Anzahl von Nutzern führt zudem dazu, dass Ausreißer bei der Bewertung einer Therapie mittels der Statusnachrichten nur einen sehr kleinen oder nahezu keinen Einfluss auf die allgemeinen Erfolgsaussichten einer Therapie für ein bestimmtes Krankheitsbild haben, da diese Ausreißer durch eine sehr große Anzahl anderer Bewertungen nahezu eliminiert werden.

Die Schritte S70 und S80 können solange wiederholt werden, bis sich alle Parameter bzw. Symptome auf ein gewünschtes Normalmaß eingependelt haben und de Therapie damit erfolgreich war.

Alternativ kann in einem Schritt S90 auch geprüft werden, ob eine Therapie abgebrochen wird, bevor sich die Symptome auf ein Normalmaß eingependelt haben. Der Abbruch einer Therapie kann beispielsweise durch einen Nutzer an der Clienteinrichtung CE veranlasst werden. Alternativ kann der Abbruch einer Therapie auch dann erfolgen, wenn beispielsweise über einen längeren Zeitraum keine Statusnachricht empfangen worden ist. In einer Ausgestaltung der Erfindung kann es bei einem Abbruch der Therapie vorgesehen sein, die zu dieser Therapie bereits empfangenen Statusnachrichten bei der Bewertung der Therapie unberücksichtigt zu lassen.

Anhand von **Fig. 3** wird der Ablauf des erfindungsgemäßen Verfahrens anhand eines konkreten Beispiels näher erläutert.

Zunächst werden in dem Schritt S10 die Parameter P1, P2 und P3 von der Servereinrichtung SE an die Clienteinrichtung CE übertragen und dort in geeigneter Weise zur Auswahl durch einen Nutzer an einer Anzeigeeinrichtung angezeigt. Die Parameter P1 bis P3 können beispielsweise Symptome sein, mit denen ein Krankheitsbild beschrieben bzw. spezifiziert werden kann.

Ein Nutzer wählt an der Clienteinrichtung CE die Parameter P1 und P2 aus. Die ausgewählten Parameter P1 und P2 werden in dem Schritt S20 von der Clienteinrichtung CE an die Servereinrichtung SE übertragen und von der Servereinrichtung SE empfangen. Das Übertragen der ausgewählten Parameter in dem Schritt S20 kann nach jeder Auswahl eines Parameters durch den Nutzer an der Clienteinrichtung CE von der Clienteinrichtung CE automatisch veranlasst werden. So wird beispielsweise nach Auswahl des Parameters P1 nur der Parameter P1 an die Servereinrichtung SE übertragen. Nach einer weiteren Auswahl des Parameters P2 wird sowohl der Parameter P1 als auch der Parameter P2 an die Servereinrichtung SE übertragen.

Nach Empfang eines oder mehrer Parameter an der Servereinrichtung SE wird durch die Servereinrichtung ein sogenanntes Matching durchgeführt. Hierzu wählt die Servereinrichtung SE in einem Schritt S31 eine Anzahl von Problembildern aus. Die Problembilder können in einer Datenbank DB gespeichert sein. In dem in Fig. 3 gezeigten Beispiel sind in der Datenbank vier Problembilder, nämlich die Problembilder PB A, PB B, PB C und PB D gespeichert, welchen jeweils eine Anzahl von Parametern zugeordnet sind. Beispielsweise sind im Problembild PB A die Parameter P1, P2 und P4 zugeordnet.

Die Auswahl in dem Schritt S31 erfolgt dermaßen, dass lediglich jene Problembilder aus der Anzahl von Problembildern selektiert werden, denen die in dem Schritt S20 übertragenen Parameter zugeordnet sind. In dem in Fig. 3 gezeigten Beispiel werden demnach die Problembilder PB A, PB B und PB C ausgewählt, denn diesen drei Problembildern sind jeweils die Parameter P1 und P2 zugeordnet. Das Problembild PB D wird nicht selektiert, denn dem Problembild PB D ist der Parameter P2 nicht zugeordnet.

Nach Auswahl der relevanten Problembilder werden in einem Schritt S32 die den ausgewählten Problembildern zugeordneten Parameter aufbereitet, um an die Clienteinrichtung CE übertragen zu werden. Die Aufbereitung der Parameter kann dergestalt sein, dass aus den den ausgewählten Problembildern zugeordneten Parametern jene Parameter ausgewählt werden, welche nicht den in dem Schritt S20 übertragenen Parametern entsprechen. In dem vorliegenden Fall wären dies für das Problembild PB A der Parameter P4, für das Problembild PB B der Parameter P5 und für das Problembild PB C die Parameter P5 und P6.

Wie in dem Beispiel nach Fig. 4 ersichtlich, ist der Parameter P5 zwei Problembildern, nämlich PB B und PB C zugeordnet. Um ein mehrfaches Übertragen von Parametern zu vermeiden, wird die Ergebnismenge der in dem Schritt S32 ermittelten Parameter derart reduziert, dass Parameter, die mehrfach in der Ergebnismenge auftreten, nur einmal berücksichtigt werden. Selbstverständlich ist eine Reduktion der Ergebnismenge nur dann erforderlich, wenn ein oder mehrere Parameter in der Ergebnismenge mehrfach vorkommen. Alternativ kann die Reduktion der Parameter auch an der Clienteinrichtung CE vorgenommen werden.

Nach der Auswahl der Parameter in dem Schritt S32 und einer gegebenenfalls erforderlichen Reduktion der Ergebnismenge werden die ausgewählten Parameter in dem Schritt S40 an die Clienteinrichtung CE übertragen und dort zur Auswahl durch einen Nutzer angezeigt. In dem vorliegenden Beispiel werden demnach die Parameter P4, P5 und P6 an die Clienteinrichtung CE übertragen. Der Nutzer der Clienteinrichtung CE kann aus den in dem ersten Auswahlschritt noch nicht ausgewählten Parameter (Parameter P3) oder aus den in dem letzten Schritt neu übertragenen Parametern (Parameter P4, P5 und P6) einen weiteren Parameter auswählten, welcher dann wiederum an die Servereinrichtung SE übertragen wird, wodurch an der Servereinrichtung SE ein erneutes Matching, wie vorstehend beschrieben, initiiert wird.

Selbstverständlich kann der Nutzer einer Clienteinrichtung CE auch die Auswahl eines bereits übertragenen Parameters rückgängig machen. Dies kann beispielsweise durch Deselektierung einer entsprechenden Checkbox erfolgen. Nach einer Deselektierung eines bereits übertragenen Parameters werden in dem Schritt S20 die verbleibenden selektierten Parameter an die Servereinrichtung SE übertragen, was in diesem Fall zur Folge hat, dass sich die Anzahl der möglichen auszuwählenden Parameter wieder vergrößern kann.

Die Schritte S20, S31, S32 und S40 können solange wiederholt werden, bis in dem Schritt S40 keine weiteren Parameter zur Übertragung vorhanden sind, oder bis der Nutzer an der Clienteinrichtung CE die Auswahl weiterer Parameter beendet. Im letzteren Fall wird das Beenden der Auswahl weiteren Parameter der Servereinrichtung SE signalisiert.

Nachdem die Auswahl beendet ist oder keine weiteren Parameter zur Auswahl mehr zur Verfügung stehen, werden durch die Servereinrichtung SE in einem Schritt S60 Lösungsvorschläge zur Lösung der Problembilder ermittelt und an die Clienteinrichtung CE übertragen. Die Auswahl der Lösungsvorschläge kann derart erfolgen, dass sämtliche zu den möglichen Problembildern zugeordneten Lösungsvorschläge selektiert werden, wobei bei der Selektion jene Problembilder berücksichtigt werden, welchen die in dem Schritt S20 empfangenen Parameter zugeordnet sind. Werden in dem in Fig. 3 gezeigten Beispiel lediglich die Parameter P1 und P2 an die Servereinrichtung SE übertragen, werden in dem Schritt S60 jene Lösungsvorschläge ausgewählt, welche den Problembildern PB A, PB B und PB C zugeordnet sind. Würde zusätzlich zu den Parametern P1 und P2 auch der Parameter P4 an die Servereinrichtung übertragen, würden lediglich jene Lösungsvorschläge selektiert, welche dem Problembild PB A zugeordnet sind.

Der Nutzer kann an der Clienteinrichtung CE nunmehr einen der möglichen Lösungsvorschläge zur Lösung des Problembildes auswählen. Der ausgewählte Lösungsvorschlag wird an die Servereinrichtung SE übertragen und dort zusammen mit einer eindeutigen Nutzerkennung gespeichert. Die weitere Verarbeitung des an die Servereinrichtung übertragenen Lösungsvorschlages sowie die Verarbeitung von Veränderungen der Parameter über die Zeit aufgrund der Anwendung des Lösungsvorschlages werden mit Bezug auf Fig. 4 näher beschrieben.

In einer weiteren Ausgestaltung der Erfindung kann es vorgesehen sein, zusätzlich zu den in dem Schritt S32 selektierten Parametern weitere Parameter zur Auswahl durch den Nutzer an die Clienteinrichtung CE zu übertragen. Beispielsweise kann ein Parameter PX, welcher keinem der in dem Schritt S31 ausgewählten Problembildern zugeordnet ist, an die Clienteinrichtung CE übertragen werden. Die Übertragung zusätzlicher Parameter kann beispielsweise durch den Nutzer an der Clienteinrichtung CE angefordert werden. Dies ist etwa dann vorteilhaft, wenn der Nutzer ein Problembild anhand eines weiteren Parameters näher spezifizieren möchte, dieser weitere Parameter aber nicht zur Auswahl angeboten wird. Die Auswahl dieses weiteren Parameters PX führt dann dazu, dass durch den Nutzer ein Problembild, etwa ein Krankheitsbild, beschrieben wird, welches der Servereinrichtung noch nicht bekannt ist, denn die Menge der möglichen Problembilder umfasst kein Problembild, welchem der Parameter PX zugeordnet ist. Nach dem Beenden der Parameterauswahl durch den Nutzer kann die Servereinrichtung ein neues Problembild generieren und in der Speichereinrichtung abspeichern, wobei dem neuen Problembild sämtliche durch den Nutzer ausgewählte Parameter zugeordnet werden. Damit wird ein im Wesentlichen selbst lernendes System bereitgestellt, welches angepasst ist, neue Problembilder mit den dazugehörigen Parametern zu erzeugen und abzuspeichern.

Solchen neuen Problembildern sind zunächst keine Lösungsvorschläge zugeordnet. Um eine einfache Zuordnung von Lösungsvorschlägen zu solchen neuen Problembildern zu ermöglichen, stellt die Servereinrichtung SE dem Nutzer eine Anzahl möglicher Lösungsvorschläge zur Auswahl zur Verfügung. Der Nutzer kann aus der Anzahl zur Verfügung gestellter Lösungsvorschläge einen Lösungsvorschlag auswählen, welcher dann dem neuen Problembild in der Datenbank zugeordnet wird.

Der Nutzer kann aber auch Teillösungen mehrerer Lösungsvorschläge auswählen, welche dann zusammen einen neuen Lösungsvorschlag bilden, welcher dem neuen Problembild zugeordnet wird.

Die serverseitige Auswahl möglicher Lösungsvorschläge für ein neues Problembild kann beispielsweise dadurch erfolgen, indem jene Lösungsvorschläge ausgewählt werden, denen zumindest teilweise die an die Servereinrichtung SE übertragenen Parameter zugeordnet sind.

Damit ist nunmehr ein neues Problembild mit den dazugehörigen Parametern und einer dazugehörigen Therapie erzeugt worden. Dieses neue Problembild und der dazugehörige Lösungsvorschlag stehen nunmehr für ein zukünftiges Erfassen von Problembildern und für ein Ermitteln von Lösungsvorschlägen zur Verfügung.

Anhand eines konkreten Beispiels aus der Medizin wird nachfolgend dieses Verhalten (Selbstlernfähigkeit des Systems) näher erläutert.

Ein Nutzer hat Bauchschmerzen und wählt dieses Symptom aus 95 möglichen Symptomen aus. Die Auswahl wird an die Servereinrichtung SE übertragen. Das erfindungsgemäße System ermittelt dann 243 unterschiedliche Krankheitsbilder, welchen das Symptom Bauchschmerzen zugeordnet ist. Diesen 243 unterschiedlichen Krankheitsbildern sind insgesamt 45 weitere Symptome zugeordnet, welche an die Clienteinrichtung CE zur weiteren Auswahl übertragen werden. Die Anzahl möglicher auszuwählender Symptome an der Clienteinrichtung hat sich somit auf insgesamt 46 Symptome reduziert.

Der Nutzer stellt nun fest, dass er auch Kopfschmerzen hat. Das Symptom Kopfschmerzen ist in der Liste der 45 weiteren Symptome enthalten. Der Nutzer wählt das Symptom Kopfschmerzen aus, welches dann an die Servereinrichtung SE übertragen wird. Die Servereinrichtung ermittelt nunmehr die Krankheitsbilder, welchen die Symptome Bauchschmerzen und Kopfschmerzen zugeordnet sind. Dadurch reduziert sich die Anzahl der 243 unterschiedlichen Krankheitsbilder auf 78 Krankheitsbilder und die Anzahl der diesen Krankheitsbildern zugeordneten Symptome reduziert sich von 45 auf 23 Symptome, welche wiederum an die Clienteinrichtung CE übertragen werden.

Nun stellt der Nutzer noch fest, dass er auch Gliederschmerzen hat und wählt dieses Symptom aus den 23 weiteren Symptomen aus. Die Auswahl führt dazu, dass sich die Anzahl möglicher Krankheitsbilder auf 17 Krankheitsbilder mit 5 weiteren Symptomen reduziert. Diese 5 Symptome werden dann zur Auswahl an die Clienteinrichtung übertragen.

Der Nutzer stellt nun weiter fest, dass er auch Durchfall hat. Das Symptom Durchfall ist aber in der Liste der noch 5 möglichen Symptome nicht enthalten. In diesem Fall kann der Nutzer die Übertragung weiterer Symptome anfordern, welche nicht den durch die Servereinrichtung ausgewählten Krankheitsbildern zugeordnet sind. Diese weiteren Symptome umfassen auch das Symptom Durchfall. Der Nutzer wählt aus diesen weiteren Symptomen das Symptom Durchfall aus, welches dann der Servereinrichtung SE übertragen wird.

Das erfindungsgemäße System erzeugt daraufhin ein neues Krankheitsbild, dem die Symptome Bauchschmerzen, Kopfschmerzen, Gliederschmerzen und Durchfall zugeordnet werden und speichert das neue Krankheitsbild in der Speichereinrichtung ab. Weil es zu diesem neuen Krankheitsbild noch keine zugeordnete Therapie bzw. Therapievorschlag gibt, werden dem Nutzer Therapievorschläge zur Auswahl bereitgestellt, denen die genannten Symptome zumindest teilweise zugeordnet sind. Beispielsweise kann ein Therapievorschlag für ein Krankheitsbild zur Auswahl bereitgestellt werden, welchem die Symptome Bauchschmerzen und Kopfschmerzen zugeordnet sind. Ferner kann ein weiterer Therapievorschlag zur Auswahl bereitgestellt werden, welcher einem Krankheitsbild mit den Symptomen Kopfschmerzen und Durchfall zugeordnet ist. Der Nutzer kann nunmehr einen der möglichen Lösungsvorschläge auswählen oder Teillösungen aus mehreren Lösungsvorschlägen selektieren. Der ausgewählte Lösungsvorschlag bzw. die ausgewählten Teillösungen werden an die Servereinrichtung übertragen und als neuer Lösungsvorschlag dem neuen Krankheitsbild zugeordnet. Damit hat das System etwas über ein neues Krankheitsbild und über einen neuen möglichen Therapievorschlag für dieses neue Krankheitsbild gelernt.

Um zu ermitteln, ob es sich bei dem neuen Therapievorschlag zu dem neuen Krankheitsbild um eine sinnvolle bzw. erfolgreiche Therapie handelt, wird dem Nutzer die Möglichkeit zur Verfügung gestellt, Veränderungen der Symptome Bauchschmerzen, Kopfschmerzen, Gliederschmerzen und Durchfall über die Zeit dem erfindungsgemäßen System mitzuteilen. Das System lernt hierbei selbstständig, welche Auswirkungen die Therapie auf das neue Krankheitsbild hat. Wenn die Therapie erfolgreich ist, werden sich die Symptome über die Zeit einem vorbestimmten Normalniveau annähern bzw. auf ein vorbestimmtes Normalniveau einpendeln, d.h., die Symptome werden verschwunden sein. Die Therapie war damit für das neue Krankheitsbild erfolgreich.

Durch die Hinterlegung der Veränderung der Symptome über die Zeit durch die Anwendung des Therapieansatzes für ein bestimmtes Krankheitsbild lernt das erfindungsgemäße System, welche Therapieansätze bei welchen Krankheitsbildern zu einer gewünschten Veränderung der Symptome führen. Auch kann das erfindungsgemäße System lernen, welche Therapieansätze zu einer unerwünschten Veränderung der Symptome über die Zeit oder gar zu neuen Symptomen (etwa Nebenwirkungen) führen. Eine unerwünschte Veränderung der Symptome oder das Auftreten neuer Symptome kann bei der Bewertung eines Therapieansatzes für ein Krankheitsbild berücksichtigt werden, wie mit Bezug auf Fig. 4 beschrieben wird.

**Fig. 4** zeigt ein Sequenzdiagramm, mit dem das Erzeugen neuer Problembilder und die Bewertung von Lösungsvorschlägen für bereits bestehende und für neue Problembilder näher erläutert werden.

Nach der Übertragung möglicher Lösungsvorschläge in einem Schritt S60 von der Servereinrichtung SE an die Clienteinrichtung CE kann der Nutzer an der Clienteinrichtung CE einen Lösungsvorschlag auswählen. Der ausgewählte Lösungsvorschlag wird in einem Schritt S65 an die Servereinrichtung SE übertragen. Die in dem Schritt S20 (vgl. Fig. 3 und Fig. 4) empfangenen Parameter bzw. das Problembild, dem die empfangenen Parameter zugeordnet sind, werden zusammen mit dem ausgewählten Lösungsvorschlag gespeichert. Zusätzlich wird im so erzeugten Datensatz eine eindeutige Nutzerkennung zugeordnet, um die Auswirkungen des Lösungsvorschlages zur Lösung des Problembildes über die Zeit für diesen Nutzer aufzeichnen zu können.

Nachdem das Problembild bzw. die Parameter und der Lösungsvorschlag für den Nutzer in dem Schritt S67 gespeichert worden sind, kann der Nutzer in regelmäßigen oder unregelmäßigen Abständen in dem Schritt S70 Statusnachrichten an die Servereinrichtung SE übertragen. Die Statusnachricht umfasst Informationen darüber, ob und wie sich die Parameter durch die Anwendung eines ausgewählten Lösungsvorschlages verändern. Beispielsweise kann mit der Statusnachricht eine Information darüber an die Servereinrichtung SE übertragen werden, dass sich ein Symptom "sehr hohes Fieber" verändert hat und nunmehr den Wert "geringes Fieber" aufweist.

In dem Schritt S81 wird die empfangene Statusnachricht ausgewertet. Die erfassten Änderungen der Parameter werden in dem Schritt S82 für das der eindeutigen Nutzerkennung zugeordnete Krankheitsbild gespeichert. Aus der Veränderung der Parameter kann das erfindungsgemäße System ermitteln, wie sich der ausgewählte Lösungsvorschlag auf das Problembild auswirkt und ob die Anwendung des Lösungsvorschlages überhaupt zu einer Lösung des Problembildes führt.

Anhand dieser Information kann in einem Schritt S83 ein Gewichtungsfaktor ermittelt werden und dem Lösungsvorschlag zugeordnet werden, wobei beim Ermitteln des Gewichtungsfaktors auch bereits dem Lösungsvorschlag zugeordnete Gewichtungsfaktoren berücksichtig werden können. Bei einer großen Anzahl von Nutzern werden dadurch die möglichen Lösungsvorschläge zu bestimmten Problembildern im Laufe der Zeit immer genauer und spezifischer, sodass im Laufe der Zeit für ein bestimmtes Problembild immer bessere Lösungsvorschläge angeboten werden können.

Mit der Statusnachricht können in dem Schritt S70 auch zusätzliche Parameter an die Servereinrichtung übertragen werden, welche, wie mit Bezug auf Fig. 3 beschrieben, keinem der ausgewählten Problembilder zugeordnet sind. Diese Parameter können beispielsweise neue Symptome sein, welche während der Anwendung einer Therapie auftreten und ein Indiz für eine Nebenwirkung der angewandten Therapie sein können.

Ein zusätzlicher Parameter kann während der Anwendung einer Therapie aber auch ein erwarteter Parameter sein, wenn für eine bestimmte Therapie während der Anwendung der Therapie ein bestimmtes Symptom erwartet wird. Beispielsweise kann bei einem bestimmten Therapieansatz vorgesehen sein, dass die Körpertemperatur bei Beginn der Therapie leicht ansteigt. Dies würde zur Folge haben, dass ein neues Symptom "erhöhte Temperatur" im Rahmen der Statusnachricht übertragen wird. Dieses zusätzliche Symptom wird dann allerdings nicht als Nebenwirkung interpretiert, sondern wirkt sich positiv auf den Gewichtungsfaktor aus, weil dieses Symptom dem erwarteten Verlauf der Therapiemaßnahme entspricht. Diese zusätzlich übertragenen Parameter können in dem Schritt S84 zu dem Problembild gespeichert werden.

Ein zusätzlich übertragener Parameter kann auch dazu führen, dass neue Problembilder entstehen, welche ebenfalls in dem Schritt S85 gespeichert werden. Zu diesen neuen Problembildern können automatisch Lösungsvorschläge erzeugt werden und den neuen Problembildern zugeordnet werden, wie beispielsweise mit Bezug auf Fig. 3 beschrieben. Diese neuen Lösungen werden in dem Schritt S86 ebenfalls gespeichert und den neuen Problembildern zugeordnet.

Das erfindungsgemäße Verfahren ist nicht darauf beschränkt, dass zu einem Krankheitsbild nur vorhandene bzw. vorgeschlagene Therapien ausgewählt werden können. Im Rahmen des Selbstlern-Mechanismus kann ein Nutzer eine neue Therapie bzw. eine neue Therapieform zu einem Krankheitsbild eingeben. Das System speichert diese neue Therapie in der Speichereinrichtung ab und verknüpft sie mit dem Krankheitsbild des Nutzers. Ist diese neue Therapie bei dem Nutzer erfolgreich kann sie bei dem gleichen Krankheitsbild auch anderen Nutzern als Therapievorschlag vorgeschlagen werden.

Zudem ist das erfindungsgemäße Verfahren angepasst neue Symptome bzw. Parameter von einem Nutzer entgegenzunehmen und zu speichern, die bisher in der Menge der Symptome noch nicht enthalten waren. Dadurch wird die Möglichkeit bereitgestellt, dass ein Nutzer aufgrund einer bestimmten Kombination von Symptomen neue Krankheitsbilder generiert bzw. beschreibt, die dem System bisher noch nicht bekannt waren. Damit kann das System sukzessive um neue Problembilder bzw. Krankheitsbilder erweitert werden. Die Möglichkeit der Erzeugung neuer Symptome bzw. Parameter und der Beschreibung neuer Krankheitsbilder bzw. Problembilder ist besonderes vorteilhaft, wenn in dem System erst eine kleine Menge an Parametern und Problembildern hinterlegt sind, was beispielsweise dann der Fall sein kann, wenn das System für eine neue Problemdomäne (z.B. Rosenzüchtung) aufgesetzt wird.

Bei der Erzeugung neuer Symptome bzw. Parameter kann es vorteilhaft sein einen Thesaurus vorzusehen, um ähnliche bereits vorhandene Parameter auszuwählen und dem Nutzer vorzuschlagen. Damit wird vermieden, dass (inhaltlich) identische Parameter mehrfach angelegt werden. Beispielsweise könnte dem Nutzer bei Eingabe des Begriffes "Bauchweh" der bereits vorhandene Parameter "Bauchschmerzen" vorgeschlagen werden. Anstelle der vorstehend beschriebenen Auswahl der Parameter über z.B. Checkboxen, kann in einer Ausgestaltung der Erfindung die Parametereingabe auch über ein Textfeld erfolgen. Auch hier kann bei Eingabe des Parameters mit Hilfe eines Thesaurus ein Abgleich mit bereits vorhandenen Parametern erfolgen. Findet das System ähnliche Parameter kann es diese zu Auswahl dem Nutzer vorschlagen.

Insgesamt wird durch die Erfindung ein Verfahren bereitgestellt, welches selbstlernend ist und aufgrund der Eingaben und Erfahrungen einer großen Anzahl von Nutzern (Schwarmintelligenz) neue Problembilder und dazugehörige Lösungsvorschläge generieren kann. Die bereits zu bestehenden Problembildern vorhandenen Lösungsvorschläge bzw. neu generierte Lösungsvorschläge werden ebenfalls aufgrund der Vielzahl von Nutzereingaben, welche etwa im Rahmen der Statusnachrichten an die Servereinrichtung übertragen werden, im Laufe der Zeit immer detaillierter und spezifischer, weil bei der Auswahl von Lösungsvorschlägen zu einem bestimmten Problembild die Auswirkungen der Lösungsvorschläge bei vorangegangenen Anwendungen der Lösungsvorschläge auf das Problembild berücksichtigt werden können. Im Laufe der Zeit kristallisieren sich dabei für bestimmte Problembilder Lösungsvorschläge heraus, mit denen diese besonders effizient gelöst werden können.

Vorstehend ist die Anwendung eines erfindungsgemäßen Verfahrens im Rahmen einer medizinischen Anwendung beschrieben worden. Selbstverständlich kann das erfindungsgemäße Verfahren bzw. das erfindungsgemäße System auch für andere Problemdomänen, etwa Probleme aus dem Automobilbereich, Probleme aus dem Telekommunikationsbereich oder dergleichen angewandt werden, soweit diese Anwendungen durch den Schutzumfang der Ansprüche abgedeckt werden.

## Patentansprüche

1. Verfahren zum Erfassen von Problembildern und zum Ermitteln von Lösungsvorschlägen in einem System umfassend zumindest eine Servereinrichtung und zumindest eine Clienteinrichtung, wobei die Clienteinrichtung über ein Kommunikationsnetzwerk mit der Servereinrichtung koppelbar ist, wobei die Servereinrichtung mit einer Speichereinrichtung gekoppelt ist, in welcher eine Anzahl von Problembildern, eine Anzahl von Parametern und eine Anzahl von Lösungen gespeichert sind, wobei den Problembildern jeweils zumindest ein Parameter und zumindest eine Lösung zugeordnet sind, wobei die einem Problembild zugeordneten Parameter das Problembild beschreiben, wobei die Problembilder Krankheitsbilder umfassen, wobei die Parameter Symptome umfassen, mit denen Krankheitsbilder beschrieben werden können, wobei die Lösungsvorschläge Therapievorschläge zur Behandlung zumindest eines Krankheitsbildes umfassen, und wobei die Servereinrichtung
a) eine Anzahl von Parametern an die Clienteinrichtung überträgt, zur Auswahl zumindest eines Parameters durch einen Nutzer,
b) den von dem Nutzer zumindest einen ausgewählten Parameter von der Clienteinrichtung empfängt,
c) aus den gespeicherten Problembildern jene Problembilder auswählt, denen die empfangenen Parameter zugeordnet sind, und aus den den ausgewählten Problembildern zugeordneten Parametern jene Parameter auswählt, welche nicht den bereits empfangenen Parametern entsprechen,
d) die ausgewählten Parameter zur Auswahl an die Clienteinrichtung überträgt;
e) die Schritte b) bis d) solange wiederholt bis in dem Schritt c) keine Parameter mehr ausgewählt werden können oder bis die Servereinrichtung von der Clienteinrichtung ein Terminierungssignal empfängt,
f) die den ausgewählten Problembildern zugeordneten Lösungen ermittelt und als Lösungsvorschläge an die Clienteinrichtung übertragt, zur Auswahl eines Lösungsvorschlages durch den Nutzer, den durch den Nutzer ausgewählten Lösungsvorschlag empfängt, den empfangenen Lösungsvorschlag zusammen mit einer Nutzerkennung in der Speichereinrichtung speichert, und dem gespeicherten Lösungsvorschlag die in dem Schritt b) empfangenen Parameter zuordnet, und
g) von der Clienteinrichtung eine Nachricht empfängt, welche eine Information darüber umfasst, ob die Anwendung des durch den Nutzer ausgewählten Lösungsvorschlages erfolgreich war,
wobei die Servereinrichtung von der Clienteinrichtung zumindest eine Statusnachricht empfängt, welche eine Information darüber umfasst, ob und wie sich die in dem Schritt b) empfangenen Parameter durch die Anwendung des ausgewählten Lösungsvorschlages verändern, wobei die Änderung der Parameter über die Zeit in der Speichereinrichtung gespeichert werden und dem ausgewählten Lösungsvorschlag zugeordnet werden, wobei basierend auf der Änderung der Parameter über die Zeit für den ausgewählten Lösungsvorschlag ein Gewichtungsfaktor ermittelt wird und dem ausgewählten Lösungsvorschlag zugeordnet wird und wobei aus der Änderung der Parameter über die Zeit ermittelt wird, ob der Lösungsvorschlag zielführend ist.

2. Verfahren nach Anspruch 1, wobei die zumindest eine Statusnachricht in vorbestimmten zeitlichen Abständen empfangen wird.

3. Verfahren nach einem der vorhergehenden Ansprüche, wobei beim Ermitteln des Gewichtungsfaktors ein bereits dem ausgewählten Lösungsvorschlag zugeordneter Gewichtungsfaktor berücksichtigt wird.

4. Verfahren nach Anspruch 3, wobei in dem Schritt f) für die an die Clienteinrichtung zu übertragenden Lösungsvorschläge eine Reihenfolge ermittelt wird, welche die Gewichtungsfaktoren der Lösungsvorschläge berücksichtigt.

5. Verfahren nach einem der vorhergehenden Ansprüche, wobei zusätzlich zu den im Schritt c) ausgewählten Parametern weitere Parameter ausgewählt werden, welche nicht den ausgewählten Problembildern zugeordnet sind, und als optionale Parameter zur Auswahl an die Clienteinrichtung übertragen werden, insbesondere dann, wenn in dem Schritt c) keine Parameter mehr ausgewählt werden können, wobei die optionalen Parameter vorzugsweise auf Anforderung durch die Clienteinrichtung ausgewählt und übertragen werden

6. Verfahren Anspruch 5, wobei bei einer Auswahl eines optionalen Parameters durch den Nutzer ein neues Problembild in der Speichereinrichtung gespeichert wird und dem neuen Problembild die empfangenen Parameter zugeordnet werden.

7. Verfahren nach Anspruch 6, wobei in dem Schritt f) Lösungen ermittelt werden, bei denen den entsprechenden Problembildern Parameter zugeordnet sind, welche zumindest teilweise von den empfangenen Parametern umfasst sind, und als Lösungsvorschläge an die Clienteinrichtung übertragen werden.

8. Verfahren nach einem der vorhergehenden Ansprüche, wobei die Statusnachricht zusätzlich eine weitere Information darüber umfasst, ob zu den bereits empfangenen Parametern weitere Parameter hinzugekommen sind und wobei die weiteren Parameter von der Servereinrichtung empfangen werden.

9. Verfahren nach Anspruch 8, wobei die weiteren Parameter dem neuen Problembild zugeordnet werden oder wobei ein neues Problembild in der Speichereinrichtung gespeichert wird und dem neuen Problembild zumindest die weiteren Parameter zugeordnet werden.

10. Verfahren nach einem der Ansprüche 8 oder 9, wobei der dem ausgewählten Lösungsvorschlag zugeordnete Gewichtungsfaktor neu ermittelt wird und/oder wobei die Information darüber, dass zu den bereits empfangenen Parametern weitere Parameter hinzugekommen sind, den neu zu ermittelnden Gewichtungsfaktor vorzugsweise negativ beeinflussen.

## Claims

1. A method of acquiring problem descriptions and of determining solution proposals in a system comprising at least one server device and at least one client device, wherein the client device may be coupled to the server device via a communications network, wherein the server device is coupled to a storage device in which a number of problem descriptions, a number of parameters, and a number of solutions are stored, wherein the problem descriptions have each at least one parameter and at least one solution associated therewith, wherein the parameters associated with a given problem description give an outline of said problem description, wherein the problem descriptions comprise clinical pictures, wherein the parameters comprise symptoms, wherein the solution proposals comprise therapeutic proposals for the treatment of at least one clinical picture, and wherein the server device
a) transmits a number of parameters to the client device, inviting a user to select at least one parameter,
b) receives the at least one parameter selected by the user from the client device,
c) selects those problem descriptions from among the stored problem descriptions with which the received parameters are associated, and selects, from among the parameters which are associated with the selected problem descriptions, those parameters which are not identical with the parameters already received,
d) transmits the selected parameters to the client device for selection by the user;
e) repeats steps b) to d) until there are no more parameters available for selection in step c) or until the server device receives a termination signal from the client device,
f) determines the solutions associated with the selected problem descriptions and transmits them as solution proposals to the client device, inviting the user to select a solution proposal, receives the solution proposal selected by the user, stores the received solution proposal, together with a user identifier, in the storage device, and associates the parameters received in step b) with the stored solution proposal, and
g) receives a message from the client device which contains information as to whether using the solution proposal selected by the user has brought success,
wherein the server device receives at least one status message from the client device which comprises information on whether, and how, the parameters received in step b) are subject to change by application of the selected solution proposal, wherein the change of the parameters over time is stored in the storage device and associated with the selected solution proposal, wherein based on the change of the parameters over time, a weighting factor is determined for the selected solution proposal and is associated with the selected solution proposal, and wherein the change of the parameters over time is used to determine whether the solution proposal is appropriate and well-targeted.

2. The method as claimed in claim 1, wherein the at least one status message is received at predetermined time intervals.

3. The method as claimed in any of the preceding claims, wherein determining the weighting factor takes into account a weighting factor which has already been associated with the selected solution proposal.

4. The method as claimed in claim 3, wherein in step f, a sequence of priority is determined for the solution proposals that are to be transmitted to the client device which takes into account the weighting factors of the solution proposals.

5. The method as claimed in any of the preceding claims, wherein in addition to the parameters selected in step c) further parameters, which are not associated with the selected problem descriptions, are selected and transmitted to the client device for the user to make a selection from, in particular when in step c) no further parameters are available for selection, said optional parameters being preferably selected and transmitted upon request by the client device.

6. The method as claimed in claim 5, wherein when an optional parameter is selected by the user, a new problem description will be stored in the storage device and the received parameters will be associated with said new problem description.

7. The method as claimed in claim 6, wherein in step f) solutions are determined in which the respective problem descriptions have parameters associated therewith which are at least partially comprised by the received parameters and which are transmitted to the client device as solution proposals.

8. The method as claimed in any of the preceding claims, wherein the status message comprises additional information as to whether further parameters have been added to the parameters already received, and wherein such further parameters are received by the server device.

9. The method as claimed in claim 8, wherein such further parameters are associated with the new problem description or wherein a new problem description will be stored in the storage device and said new problem description will have at least such further parameters associated therewith.

10. The method as claimed in any one of claims 8 or 9, wherein the weighting factor associated with the selected solution proposal will be redetermined and/or wherein an information on the fact that further parameters have been added to the parameters already received will preferably negatively influence the weighting factor that is to be redetermined.

## Revendications

1. Procédé destiné à saisir des tableaux de problème et à déterminer des propositions de solution dans un système comprenant au moins un dispositif serveur et au moins un dispositif client, le dispositif client pouvant être couplé au dispositif serveur par l'intermédiaire d'un réseau de communication, le dispositif serveur étant couplé à un dispositif mémoire dans lequel sont stockés un nombre donné de tableaux de problème, un nombre donné de paramètres et un nombre donné de solutions, au moins un paramètre et au moins une solution étant respectivement attribués aux tableaux de problème, les paramètres attribués à un tableau de problème décrivant ledit tableau de problème, les tableaux de problème comprenant des tableaux cliniques, les paramètres comprenant des symptômes permettant de décrire des tableaux cliniques, les propositions de solution comprenant des propositions thérapeutiques destinées à traiter au moins un tableau clinique, et dans lequel procédé le dispositif serveur
a) transmet un nombre donné de paramètres au dispositif client afin qu'au moins un paramètre soit sélectionné par un utilisateur,
b) reçoit du dispositif client ledit au moins un paramètre sélectionné par l'utilisateur,
c) sélectionne parmi les tableaux de problème stockés les seuls tableaux de problème auxquels sont attribués les paramètres reçus et sélectionne parmi les paramètres attribués aux tableaux de problème sélectionnés les seuls paramètres qui ne correspondent pas aux paramètres déjà reçus,
d) transmet au dispositif client les paramètres sélectionnés en vue de les soumettre à une nouvelle sélection ;
e) répète les étapes b) à d) jusqu'à ce qu'il n'y ait plus aucun paramètre à sélectionner à l'étape c) ou jusqu'à ce que le dispositif serveur reçoive du dispositif client un signal de terminaison,
f) détermine les solutions attribuées aux tableaux de problème sélectionnés et les transmet au dispositif client en tant que propositions de solution afin que l'utilisateur puisse sélectionner une proposition de solution, reçoit la proposition de solution sélectionnée par l'utilisateur, stocke dans le dispositif mémoire, ensemble avec un identifiant de l'utilisateur, la proposition de solution reçue et attribue les paramètres reçus à l'étape b) à la proposition de solution stockée, et
g) reçoit du dispositif client un message qui comprend une information indiquant si l'utilisation de la proposition de solution sélectionnée par l'utilisateur s'est avérée efficace,
le dispositif serveur recevant du dispositif client au moins un message d'état qui comprend une information indiquant si et comment les paramètres reçus à l'étape b) se modifient suite à l'utilisation de la proposition de solution sélectionnée, le changement des paramètres dans le temps étant stocké dans le dispositif mémoire et étant attribué à la proposition de solution sélectionnée, un facteur de pondération étant déterminé sur la base du changement des paramètres dans le temps obtenu pour la proposition de solution sélectionnée et étant attribué à ladite proposition de solution sélectionnée et à partir dudit changement des paramètres dans le temps étant déterminé si la proposition de solution est appropriée.

2. Procédé selon la revendication 1 lors duquel ledit au moins un message d'état est reçu à des intervalles de temps prédéfinis.

3. Procédé selon l'une quelconque des revendications précédentes lors duquel un facteur de pondération déjà attribué à la proposition de solution sélectionnée est pris en considération lors de la détermination du facteur de pondération.

4. Procédé selon la revendication 3 lors duquel une succession qui prend en considération les facteurs de pondération des propositions de solution est déterminée à l'étape f) pour les propositions de solution à transmettre au dispositif client.

5. Procédé selon l'une quelconque des revendications précédentes lors duquel, en plus des paramètres sélectionnés à l'étape c), sont sélectionnés d'autres paramètres qui ne sont pas attribués aux tableaux de problème et qui sont transmis en tant que paramètres optionnels au dispositif client dans le but d'être soumis à une sélection, et ce en particulier lorsqu'aucun paramètre ne peut plus être sélectionné à l'étape c), les paramètres optionnels étant de préférence sélectionnés et transmis à la demande du dispositif client.

6. Procédé selon la revendication 5 lors duquel un nouveau tableau de problème est stocké dans le dispositif mémoire lorsqu'un paramètre optionnel est sélectionné par l'utilisateur et les paramètres reçus sont attribués au nouveau tableau de problème.

7. Procédé selon la revendication 6 lors duquel à l'étape f) sont déterminées des solutions dans lesquelles des paramètres compris au moins en partie dans les paramètres reçus sont attribués aux tableaux de problème respectifs et sont transmis au dispositif client en tant que propositions de solution.

8. Procédé selon l'une quelconque des revendications précédentes lors duquel le message d'état comprend en outre une autre information indiquant si d'autres paramètres sont venus s'ajouter aux paramètres déjà reçus et lors duquel lesdits autres paramètres sont reçus par le dispositif serveur.

9. Procédé selon la revendication 8 lors duquel les autres paramètres sont attribués au nouveau tableau de problème ou lors duquel un nouveau tableau de problème est stocké dans le dispositif mémoire et au moins les autres paramètres sont attribués audit nouveau tableau de problème.

10. Procédé selon l'une des revendications 8 ou 9 lors duquel le facteur de pondération attribué à la proposition de solution sélectionnée est redéterminé et/ou lors duquel l'information indiquant que d'autres paramètres sont venus s'ajouter aux paramètres déjà reçus influence de préférence de manière négative le facteur de pondération qui doit être redéterminé.
